# EUROPÄISCHE PATENTSCHRIFT

(11) **EP 1 993 669 B1**
(45) Veröffentlichungstag und Bekanntmachung des Hinweises auf die Patenterteilung: **29.12.2010**
(21) Anmeldenummer: 07711909.7
(22) Anmeldetag: 12.03.2007
(51) Int. Cl.: A61N 5/10, B01L 3/00

(54) **BESTRAHLUNGSVERIFIKATIONSVORRICHTUNG FÜR STRAHLENTHERAPIEANLAGEN UND VERFAHREN ZUR HANDHABUNG DERSELBEN**
IRRADIATION VERIFICATION DEVICE FOR RADIOTHERAPY INSTALLATIONS, AND METHOD FOR HANDLING THEREOF
DISPOSITIF DE VÉRIFICATION D'IRRADIATION POUR DES INSTALLATIONS DE RADIOTHÉRAPIE ET SON PROCÉDÉ DE MANIPULATION

(30) Priorität: 13.03.2006 DE 102006011828; 06.07.2006 DE 102006031496
(43) Veröffentlichungstag der Anmeldung: 26.11.2008
(73) Patentinhaber: GSI Helmholtzzentrum für Schwerionenforschung GmbH, 64291 Darmstadt (DE)
(72) Erfinder: VON NEUBECK, Cläre, Hanna, 61440 Oberursel (DE); KRAFT-WEYRATHER, Wilma, 64291 Darmstadt (DE); GÜBITZ, Carola, Sabine, 65779 Kelkheim (DE)
(74) Vertreter: Forstmeyer, Dietmar
(86) Internationale Anmeldenummer: PCT/EP2007/002156
(87) Internationale Veröffentlichungsnummer: WO 2007/104520

(56) Entgegenhaltungen:
- WO-A-98/53046
- DE-A1- 4 132 379
- US-A- 5 837 115
- A. MITAROFF, W. KRAFT-WEYRATHER, O. B. GEISS: "Biological verification of heavy ion treatment planning" RADIAT ENVIRON BIOPHY, 1998, - 1998 Seiten 47-51, XP002433356
- B. G. WOUTERS ET AL: "Measurements of Relative Biological Effectiveness of the 70 Mev Proton beam at TRIUMF" RADIATION RESEARCH, 1996, - 1996 Seiten 159-170, XP009083658

## Beschreibung

Die Erfindung betrifft eine Bestrahlungsverifikätionsvorrichtung für Strahlentherapieanlagen, wobei die Bestrahlungsverifikationsvorrichtung lebendes Zellenmaterial aufweist, dass in einem Behälter mit einem Einsatz auf Zellträgern des Einsatzes örtlich in den drei Raumkoordinaten x, y und z fixiert ist. Eine derartige Bestrahlungsverifikationsvorrichtung ist aus der Druckschrift von A. Mitaroff u.a. mit dem Titel "Biological Verification of Heavy Ion Treatment Planning", Radiat Environ Biophys (1998) 37, S. 27-52 bekannt. Bestrahlungsverifikationsvorrichtungen werden eingesetzt, um ein Strahlungskonzept auf seine Wirksamkeit zu prüfen, bevor eine Strahlentherapieanlage zur Behandlung von Menschen freigegeben wird.

Bei der in der obigen Druckschrift beschriebenen Bestrahlungsverifikationsvorrichtung wird das lebende Zellmaterial auf Plastikobjektträger von 9 mm Breite, 53 mm Länge und 1 mm Dicke aufgebracht. Diese mit lebendem Zellmaterial beschichteten Objektträger werden durch zwei geschlitzte Platten, wobei die Schlitze in den Platten der Breite und Dicke der Plastikobjektträger entsprechen und Raum für den Zellbelag lassen, in einem definierten Abstand waagerecht zueinander stehend geschoben und mit einer dritten durchgängig geschlitzten Platte fixiert.

Ein derart präparierter Einsatz mit lebendem Zellmaterial beschichteten Objektträgern wird in einen zylindrischen Behälter gestellt und beispielsweise als so genanntes "Kopfphantom" in eine Bestrahlungsposition verbracht, die der Bestrahlungsposition beispielsweise eines menschlichen Kopfes zur Behandlung eines Tumors entspricht. Zur Verifikation wird das Strahlungsprogramm eingegeben und das "Kopfphantom" entsprechend bestrahlt, wobei anschließend die Position der abgestorbenen Zellen in dem Volumen des Behälters mit Hilfe der Objektträger bestimmt wird.

Das bekannte Modell hat den Nachteil, dass für eine genaue Abgrenzung die Objektträger mit 9 mm Breite und 52 mm Höhe eine Auflösung orthogonal zum Strahl in x- und y-Richtung liefern, die keine hohe Auflösung zulässt. Dadurch kann der Übergangsbereich zwischen bestrahltem Gewebe und unbestrahltem Gewebe nur relativ ungenau in den Randbereichen oder Übergangsbereichen angegeben werden. In Bestrahlungsrichtung können keine lebenden Zellen aufgebracht werden, da hier nur die Querseite von 1 mm der Objektträger zur Verfügung steht. Somit ergibt sich für die Auflösung in x-Richtung eine Auflösung von 9 mm und in y-Richtung eine Auflösung von 52 mm, zumal die Objektträger nicht unterteilt werden können.

Darüber hinaus ist von Nachteil, dass der Zellträger aus einem Objektträger vor und nach der Bestrahlung durch zwei Schlitze der Halteplatten gezogen wird, so dass es zu unkontrollierten Beschädigungen des Zellmaterials kommen kann, was die Ergebnisse verfälscht.

Aus der Druckschrift W. G. Wouters "Mesurements of Relative Biological Effectiveness of the 70 MeV Proton Beam at TRIUMF using Chinese Hamster V 79 Cells and the High Precision Cell Sauter Assay", Radiation Research Vol. 146, S. 159-170 (1996), Radiation Research Society, ist eine Bestrahlungsverifikationsvorrichtung bekannt, welche die lebenden Zellen in einem Nahrungsgel unterbringt und damit ein Hohlrohr auffüllt, dass strahlendurchlässig ist. Während der Bestrahlung wird die Gelmasse im Hohlrohr auf 4° Celsius heruntergekühlt, so dass das Gel aushärtet. Nach der Bestrahlung kann die Gelmasse dem Rohr entnommem werden und in Scheiben getrennt werden, und es kann die örtliche Verteilung der aktivierbaren lebenden Zellen gegenüber dem abgestorbenen Material durch Rekultivierung festgestellt werden. Diese Bestrahlungsverifikationsvorrichtung würde, wenn man mehrere Rohre nebeneinander stapelt eine Auflösung in x- und y-Richtung von lediglich 12 mm bringen, da eine ausreichende Gelmasse zur Entwicklung und zum Überleben der Zellen erforderlich ist und der Durchmesser der Hohlzylinder nicht weiter verkleinert werden kann.

Zwar kann durch Auftrennen des Gels in 2 mm dünne Scheiben eine hohe Auflösung in z-Richtung erreicht werden, aber es die experimentelle Durchführung zeigt, dass das ein hohes Risiko besteht, dass die Scheiben ungleichmäßig breit geschnitten werden, so dass in diesem Falle eine genaue Ortsbestimmung der Probenposition mit zugehörigen Dosenbestimmung nicht mehr möglich ist.

Während somit der Bestrahlungsverifikationsvorrichtung nach_ W. G. Wouters ein gutes Tiefenprofil liefert, wenn die Gelproben entsprechend präzise entnommen werden, liefert die Bestrahlungsverifikationsvorrichtung nach A. Mitaroff u.a. eine zusätzliche Information über die Verteilung in x- und in y-Richtung, jedoch mit geringerem Auflösungspotential.

Aufgabe der Erfindung ist es, eine Bestrahlungsverifikationsvorrichtung für Strahlentherapieanlagen zu schaffen, deren Auflösungsvermögen größer ist und damit auch eine zuverlässigere Überprüfung eines geplanten Bestrahlungskonzepts ermöglicht. Ferner soll die Handhabbarkeit bei verbesserter Auflösung erleichtert werden, um schnellere Ergebnisse zu liefern

Diese Aufgabe wird mit dem Gegenstand der unabhängigen Ansprüche gelöst. Vorteilhafte Weiterbildungen der Erfindung ergeben sich aus den abhängigen Ansprüchen.

Erfindungsgemäß wird eine Bestrahlungsverifikationsvorrichtung angegeben, die lebendes Zellmaterial aufweist, das auf Zellträgern in einem Behälter mit einem Einsatz örtlich in den drei Raumkoordinaten x, y und z fixiert ist. Die Zellträger sind zwischen Zellträgerhaltern angeordnet. Zur Bestrahlungsverifikation ist die z-Raumkoordinate der Bestrahlungsverifikationsvorrichtung in Richtung der Strahlachse Z justiert, so dass nach der Bestrahlung Bereiche mit abgestorbenem Zellmaterial von Bereichen mit noch aktivem Zellmaterial der Bestrahlungsverifikationsvorrichtung in Raumkoordinaten unter Bezug auf ein Bestrahlungskonzept lokalisierbar sind.

Der Behälter mit dem Einsatz für die Zellträger sowie die Zellträgerhalter weisen ein strahlendurchlässiges Material auf. Die Zellträgerhalter weisen eine Bodenplatte und eine Deckplatte auf, zwischen denen die Zellträger orthogonal zu den Halteplatten angeordnet sind. Die Halteplatten haben aufeinander ausgerichtete Blindlöcher, in denen die Enden der Zellträger angeordnet sind. Die Zellträger sind Kreiszylindrische Vollstäbe, auf deren Mantelflächen das Zellmaterial fixiert bzw. aufgewachsen ist.

Eine derartige Bestrahlungsverifikationsvorrichtung hat den Vorteil, dass sie schnell und einfach zusammengebaut werden kann, sobald die stabförmigen Zellträger auf ihren Mantelflächen lebendes Zellmaterial aufweisen. Außerdem können beim Zusammenbau, nämlich beim Einstecken der Vollstäbe in Blindlöcher der Bodenplatte und beim Aufsetzen der Deckplatte auf die Zellstäbe unter Einführen der freien Enden der Zellstäbe in die Blindlöcher der Deckplatte, die frei bleibenden Mantelflächen mit dem Zellmaterial nicht beschädigt werden, wie es bei der herkömmlichen Objektträgerlösung der Fall ist. Schließlich können die Vollstäbe zwischen den Halteplatten mit ihren Blindlöchern derart dicht gestapelt werden, dass eine hohe Auflösung sowohl in Strahlrichtung bzw. in der z-Koordinate, als auch in der horizontalen x-Koordinate erreicht werden.

Für die vertikale y-Koordinate kann eine besonders hohe Auflösung erreicht werden, indem nach dem Bestrahlen die Vollstäbe in dünne Scheiben, deren Ränder mit Zellmaterialien belegt sind, getrennt werden. Für Zellträger kann ein zellverträgliches Material vorgesehen werden, insbesondere mit einer wassernahen Dichte von etwa 1 g/cm³ und insbesondere frei von Metallen und Elementen höherer Ordnungszahl als Sauerstoff. Dazu sind vorzugsweise die Zellträger aus einem Polystyrol-Material, das den Vorteil hat, dass es sich präzise und leicht in einzelne Scheiben mit geringem Abstand und Aufwand auftrennen lässt.

Die hohe Packungsdichte der beiden Halteplatten wird dadurch verstärkt, dass in einer bevorzugten Ausführungsform der Erfindung die Zellträger versetzt zueinander zwischen den Halteplatten in Reihen angeordnet sind. Dabei wechseln Reihen mit einer geraden Anzahl von Vollstäben bzw. Zellträgern mit Reihen, die eine ungerade Anzahl von Vollstäben aufweisen ab. Wenn gleichzeitig auch ein minimaler Abstand zwischen den Stäben eingehalten wird, dann ist die Auflösung in Bestrahlungsrichtung und in der horizontalen x-Richtung gegenüber den bisherigen Bestrahlungsverifikationsvorrichtungen deutlich verbessert, zumal für ein großes Untersuchungsvolumen alle drei Raumrichtungen überprüft werden können.

Um ein großes Bestrahlungsvolumen zu erfassen, das etwa dem Kopf eines Menschen entspricht und deshalb auch "Kopfphantom" genannt wird, werden vorzugsweise Zellträger von einer Länge 1 zwischen 25 mm ≤ l ≤ 75 mm, vorzugsweise eine Länge 1 von etwa 50 mm, eingesetzt. Die Messlänge bzw. die Mantelfläche dieser Stäbe, die mit lebenden Zellen belegt ist, ist jedoch geringer, da beispielsweise ein solcher Stab etwa 10 mm in einem Blindloch in einer Bodenplatte fixiert wird, und auch für das Fixieren des frei stehenden Endes in einer Deckplatte wiederum ein Teil der Länge beispielsweise 5 mm in einem Blindloch einer derartigen Deckplatte verschwinden.

Für die Auflösung in Bestrahlungsrichtung Z, also einer Tiefenmessung mit Hilfe der Bestrahlungsverifikationsvorrichtung und für eine horizontalen Auslenkung X des Strahls in x-Richtung ist der Durchmesser d der Vollstäbe entscheidend. Dieser Durchmesser d in Millimetern beträgt 1,0 mm ≤ d S 5 mm, insbesondere 1,5 mm ≤ d S 5 mm, vorzugsweise beträgt der Durchmesser etwa 3 mm.

Die Auflösung in Strahlrichtung und die Auflösung der horizontalen Ablenkung des Strahls hängt auch von dem minimalen Abstand a zwischen den Zellträgern in Millimetern ab, wobei dieser Abstand a zwischen 0,5 mm ≤ a ≤ 5 mm liegt, vorzugsweise beträgt der Abstand a in Millimetern 2,5 mm.

Um die Vollstäbe aus Polystyrol nicht durch einen hohen Einspanndruck zwischen den beiden Halteplatten zu belasten oder zu beschädigen, sind an den Randseiten der Halteplatten Vorsprünge vorgesehen, die sich auf Abstandshalter zwischen den Halteplatten abstützen, so dass die stabförmigen Zellträger in vorteilhafter Weise mit einer losen Passung in der Bondenplatte und in der Deckplatte positioniert sind. Zusätzlich kann die Tiefe der Blindlöcher in der Bodenplatte größer sein als die Tiefe der Blindlöcher in den Deckplatten, so dass die in die Bodenplatte eingeführten Stäbe trotz loser Passung eine genauere Ausrichtung in Richtung auf die Deckplatte erfahren.

Um das Einführen der säulen- bzw. stabförmigen Zellträger in die Blindlöcher zu erleichtern, weisen die Blindlöcher der Halteplatten eine kegelförmige Ansenkung ihrer Außenränder auf. Durch diese Ansenkung gleiten die stabförmigen Zellträger ohne jede Beschädigung in die vorgesehenen Blindlöcher hinein. Vorzugsweise weist die Bestrahlungsverifikationsvorrichtung eine Anzahl S von Zellträgern als Vollstäbe von 150 ≤ S ≤ 900 und insbesondere von 400 ≤ S ≤ 900 auf.

Die lebenden Zellen auf den Mantelflächen der Vollstäbe müssen ernährt werden und brauchen wozu Gas(e) im Nährmedium gelöst sein kann (können), beispielsweise Sauerstoff und/oder Kohlendioxid. Deshalb ist in einer weiteren bevorzugten Ausführungsform eine der beiden Halteplatten mit Durchgangslöchern zusätzlich zu den Blindlöchern versehen, wobei in bevorzugter Ausführungsform der Erfindung immer in dem Zentrum eines Dreiecks, das aus drei benachbarten Blindlöchern gebildet wird, ein Durchgangsloch vorgesehen ist, um das Durchströmen beispielsweise von Nährstofflösung zu ermöglichen.

Um eine genaue Zuordnung der Positionen der stabförmigen Zellträger zu ermöglichen, sind vorzugsweise Markierungslinien oder durchgehende Positionierungsnummern für die Stabpositionen auf der Bodenplatte und/oder auf der Deckplatte vorgesehen. Außerdem weist der Einsatz in Verlängerung der Abstandshalter für die Halteplatten weitere Verlängerungen in Richtung auf den Boden des Behälters auf, die als Standbeine dienen.

Da bei einer derartigen biologischen Bestrahlungsverifikationsvorrichtung die Nährflüssigkeit an den Stäben in Bewegung zu halten ist, weist der Behälter im Bodenbereich zwischen der Bodenplatte des Einsatzes und dem Boden des Behälters mindestens ein Rührsystem auf, das in einem Zwischenraum angeordnet ist, welcher durch die Standbeine geschaffen ist. Der Behälter, der für die Montage des Einsatzes nach oben offen ist weist zusätzlich mindestens eine Einlassöffnung und eine Auslassöffnung für Medien wie Nährflüssigkeit und/oder Sauerstoff auf. Über die Medienöffnungen kann der Innenraum des Behälters mit entsprechenden Nährlösungen bzw. mit Sauerstoff versorgt werden, während die Beschickungsöffnung auf der Oberseite nach einem Positionieren des Einsatzes abgedeckt ist.

Eine bevorzugte Verwendung der erfindungsgemäßen Bestrahlungsverifikationsvorrichtung sind Strahlentherapieanlagen, wie Röntgenstrahltherapieanlagen oder Protonenstrahltherapieanlagen, und insbesondere ist die erfindungsgemäße Bestrahlungsverifikationsvorrichtungen für Heliumionenstrahltherapieanlagen und/oder Kohlenstoffionenstrahltherapieanlagen entwickelt worden.

Ein Verfahren zur Handhabung der erfindungsgemäßen Bestrahlungsverifikationsvorrichtung weist die nachfolgenden Verfahrensschritte auf. Zunächst werden Mantelflächen von stabförmigen Zellträgern mit aktivem lebendem Zellmaterial belegt. Anschließend folgt ein Zusammenbau eines Einsatzes für einen Behälters der Bestrahlungsverifikationsvorrichtung. Dazu werden die Zellträger mit lebendem Zellmaterial zwischen zwei Halteplatten des Einsatzes und unter Sichern des zusammengebauten Einsatzes über Abstandshalter zusammengefügt. Dabei kommt dem Zusammenbau und dem späteren Auseinandernehmen eine Markierung der Bodenplatte zugute und auch das Vorsehen tieferer Blindlöcher in der Bodenplatte, in denen die stabförmigen Zellträger sicher ausgerichtet und aufgestellt werden können.

Dann erfolgt das Einbringen des kompletten Einsatzes in den zwischenzeitlich mit Nährflüssigkeit aufgefüllten Behälter. Außerdem wird Sauerstoff über eine Einlassöffnung in den Behälter zugeführt und falls erforderlich auch noch zusätzliche frische Nährlösung. Danach erfolgt das Ausrichten der x-, y-, und z-Raumkoordinaten der Bestrahlungsverifikationsvorrichtung zur Strahlrichtung Z der Bestrahlungsanlage. Nun kann das geplante Bestrahlungskonzept unter gleichzeitiger oder nachträglicher Temperierung, beispielsweise Kühlung oder Wärmung der Nährflüssigkeit, um die Ergebnisse zu konservieren, durchgeführt werden. Nach dem Ausbau der Zellträger aus dem Einsatz werden diese in Scheiben aufgetrennt. Das noch lebende Zellmaterial auf den Rändern der Scheiben wird detektiert. Abschließend können die Ergebnisse in Bezug auf die gewählten analytischen Endpunkte mit dem Bestrahlungsplan verglichen werden.

Dieses Handhabungsverfahren zeigt deutlich den Vorteil dieser Bestrahlungsverifikationsvorrichtung gegenüber herkömmlichen Techniken zur biologischen Verifikation von Strahlentherapieanlagen.

Die Erfindung wird nun anhand der beigefügten Figuren näher erläutert.
- Figur 1: zeigt eine schematische perspektivische Ansicht eines Einsatzes in Zusammenbauposition einer Bestrahlungs- verifikationsvorrichtung gemäß einer Ausführungsform der Erfindung;
- Figur 2: zeigt eine schematische perspektivische Ansicht einer Bodenplatte des Einsatzes gemäß Figur 1;
- Figur 3: zeigt eine schematische perspektivische Ansicht einer Deckplatte des Einsatzes gemäß Figur 1;
- Figur 4: zeigt eine schematische perspektivische Ansicht des Einsatzes gemäß Figur 1 in Bestrahlungsposition;
- Figur 5: zeigt ein geplantes Strahlungskonzept für zwei Be- strahlungsverifikations-Experimente;
- Figur 6: zeigt eine schematische Draufsicht auf einen Behälter der Bestrahlungsverifikationsvorrichtung mit Ergeb- nissymbolen für Gruppen von Zellträgern eines ersten Bestrahlungs-Experiments;
- Figur 7: zeigt eine schematische Draufsicht auf den Behälter der Bestrahlungsverifikationsvorrichtung mit Ergeb- nissymbolen für Gruppen von Zellträgern eine zweiten Bestrahlungs-Experiments;
- Figur 8: zeigt eine schematische Draufsicht auf den Behälter der Bestrahlungsverifikationsvorrichtung mit gemit- telten Ergebnissymbolen für Gruppen von Zellträgern;
- Figur 9: zeigt einen Plan der Lage von Verifikationsmesslinien A bis D durch die Bestrahlungsverifikationsvorrich- tung;
- Figur 10: zeigt Mittelwerte von Experimenten entlang der Veri- fikationsmesslinie A-A in Figur 9;
- Figur 11: zeigt Mittelwerte von Experimenten entlang der Veri- fikationsmesslinie B-B in Figur 9;
- Figur 12: zeigt Mittelwerte von Experimenten entlang der Veri- fikationsmesslinie C-C in Figur 9;
- Figur 13: zeigt Mittelwerte von Experimenten entlang der Veri- fikationsmesslinie D-D in Figur 9;

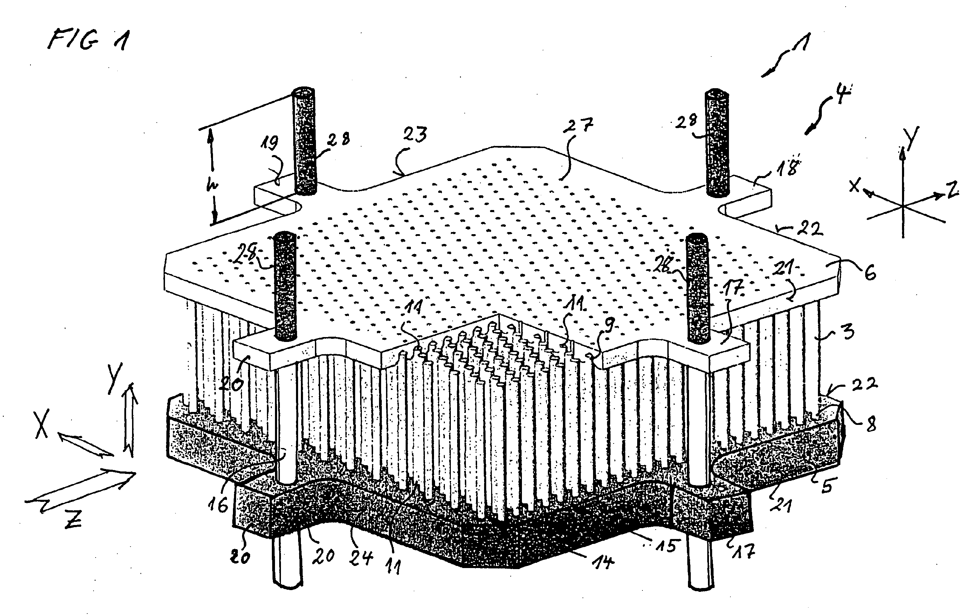

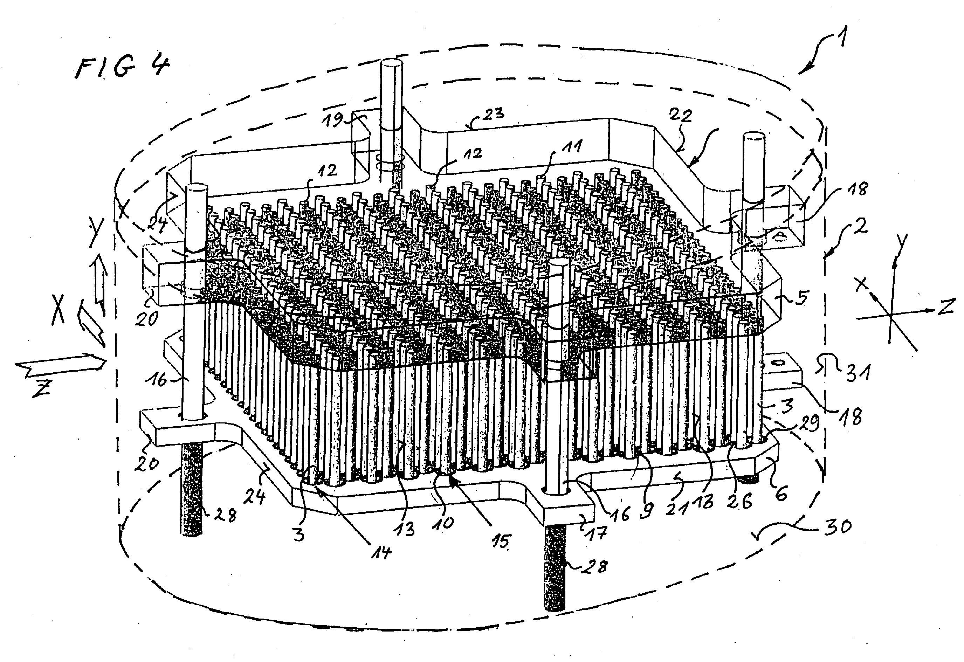

Figur 1 zeigt eine schematische perspektivische Ansicht eines Einsatzes 4 hier in Zusammenbauposition einer Bestrahlungsverifikationsvorrichtung 1 gemäß einer Ausführungsform der Erfindung. Die hier gezeigte Zusammenbauposition unterscheidet sich von der in Figur 4 gezeigten Bestrahlungsposition dadurch, dass der Einsatz 4 in den hier nicht gezeigten zylindrischen Behälter der Bestrahlungsverifikationsvorrichtung 1 um 180° gedreht eingeführt wird, so dass die hier gezeigte Deckplatte 6 in Figur 4 zur unteren Halteplatte wird, und die mit Zusammenbau in Figur 1 gezeigte Bodenplatte 5 in Figur 4 in der Bestrahlungsposition eine obere Halteplatte ausbildet.

Die hier gezeigte Bodenplatte 5 in der Zusammenbauposition hat eine Dicke von etwa 12 mm und weist Reihen von Blindbohrungen bzw. Blindlöcher 8 auf, die mit einer Schrittweite von 5,5 mm nebeneinander in Reihen 14 und 15 angeordnet sind. Die Tiefe der Blindbohrungen 8 in der Bodenplatte 5 ist tiefer als die Blindbohrungen 9 in der Deckplatte 6. In dieser Ausführungsform der Erfindung weisen die Blindbohrungen 8 und 9 einen Durchmesser von 3,2 mm auf, während die Kreiszylindrische stabförmigen Zellträger 3 einen Durchmesser von 3 mm haben. Dazu sind die Vollstäbe 12 der Zellträger 3 aus Polystyrol aufgebaut und weisen auf ihrer Mantelfläche 13 lebende Zellkolonien auf. Die Anzahl der Stäbe ist in dieser Ausführungsform der Erfindung 587.

Dieses Zellmaterial 12 bedeckt die Mantelfläche 13 und erstreckt sich über die gesamte Länge 1 der stabförmigen Zellträger 3 bis auf das eine Ende 10, das mit 10 mm in einer Spielpassung in Blindlöchern 8 der Bodenplatte 5 eingebracht ist. Von der Stablänge 1 von 50 mm sind darüber hinaus 3,5 mm in Blindlöchern 9 der Deckplatte 6 angeordnet, so dass eine Nettostablänge, deren Mantelfläche 13 mit Zellmaterial bedeckt ist, von 36,5 mm für die biologische Verifikation eines geplanten Bestrahlungskonzepts vertikal zur Strahlachse zur Verfügung steht. Die nur 5 mm dicke Deckplatte weist neben den Blindlöchern 9 zur Aufnahme der Zellträger 3 Durchgangslöcher 27 auf, durch die Sauerstoff und Nährlösung in den Bereich der Zellkulturen strömen kann. Deshalb wird die hier gezeigte Deckplatte 6 in der Bestrahlungsposition die untere Halteplattenposition einnehmen.

Die über die Deckplatte 6 in Figur 1 hinausragenden Stücke dienen dann als Standbeinen 28 in dem hier nicht gezeigten Behälter der Bestrahlungsverifikationsvorrichtung 1. Die Höhe h des Standbeins 28, mit der das Standbein 28 über die Halteplatte 6 hinausragt, ist groß genug, um in der Bestrahlungsposition im Bodenbereich einen Zwischenraum zu schaffen, in dem ein Rührsystem arbeiten kann, um eine Nährlösung in Bewegung zu halten. Die Durchgangslöcher 27 der Deckplatte 6 stehen damit direkt im Wirkungsbereich des Rührsystems, wobei neben Sauerstoff auch Nährflüssigkeit durch die Durchgangslöcher 27 strömt. Die Standbeine 28 sind auf Vorsprüngen 17-20 der Halteplatten 5 und 6 angeordnet, wobei auf den Randseiten 21-24 Abstandshalter 16 angeordnet sind, die einen derartigen Abstand aufrechterhalten, so dass die Vollstäbe 12 in den Blindlöchern 8 und 9 eine Spielpassung beibehalten und kein Druck auf die Enden 10 und 11 der Zellträger 3 ausüben, wobei die Zellträger einen Durchmesser zwischen 1,0 mm und 5,0 und vorzugsweise etwa 3 mm aufweisen.

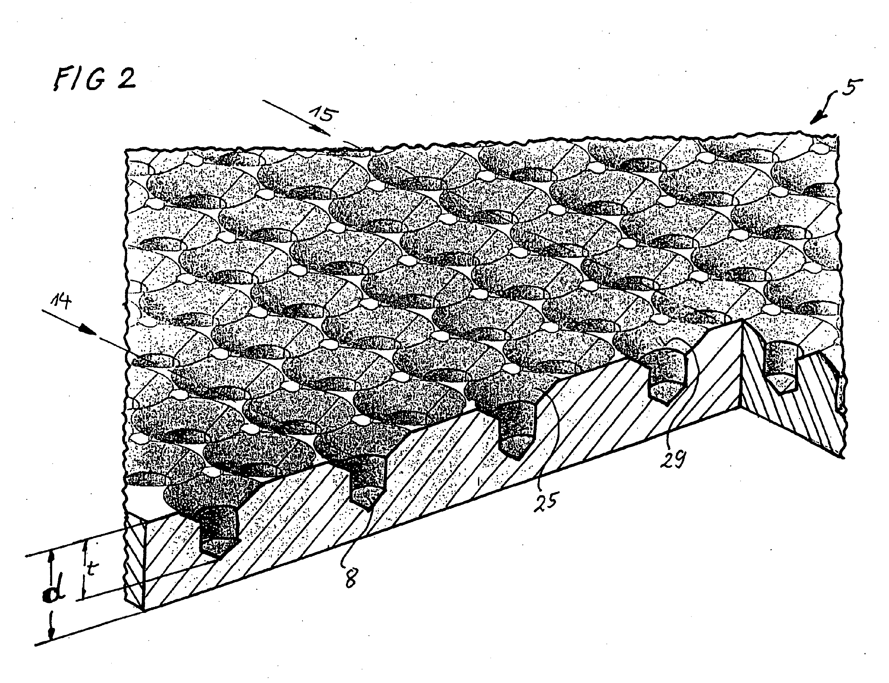

Figur 2 zeigt eine schematische perspektivische Ansicht einer Bodenplatte 5 des Einsatzes 4 gemäß Figur 1. In der Bodenplatte 5 mit einer Dicke d von 12 mm ± 1 mm sind Blindlöcher 8 oder auch Sacklöcher eingelassen worden, die eine Tiefe t von 10 mm erreichen. Bei einer Durchmessertoleranz von etwa 0,2 mm können die Zellträger orthogonal zur Bodenplatte 5 in den Blindlöchern ausgerichtet stehen, bis auch die Abstandshalter montiert sind, und die Deckplatte aufgesetzt werden kann.

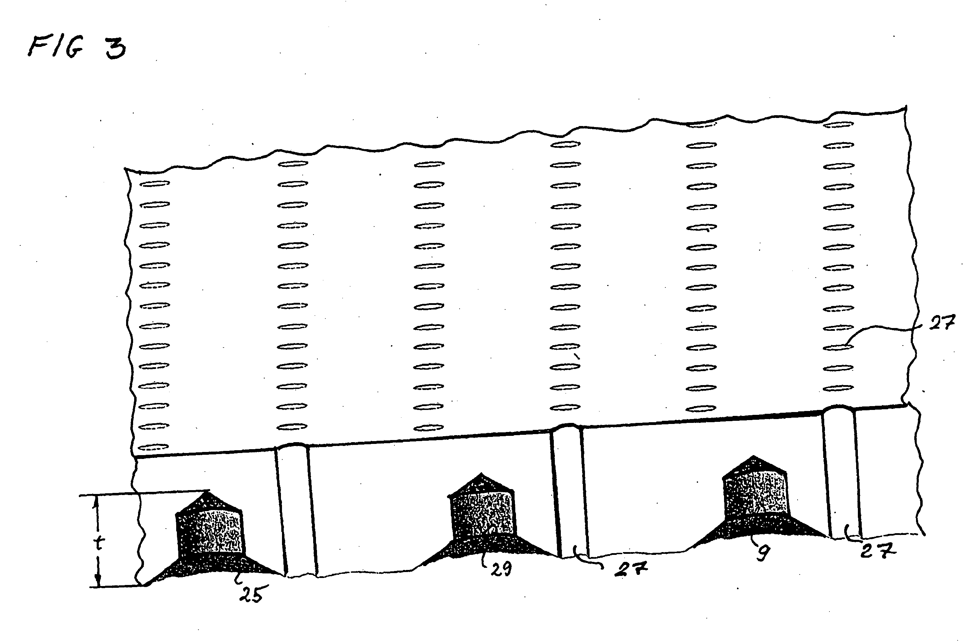

Figur 3 zeigt eine schematische perspektivische Ansicht einer Deckplatte 6 des Einsatzes 4 gemäß Figur 1. Die Blindlöcher 9 der Deckplatte 6 sind auf die Blindbohrungen 8 der Bodenplatte 5 wie sie in Figur 2 gezeigt werden ausrichtbar. Damit die stabförmigen Zellträger in die Blindlöcher 9 der Deckplatte 5 beim Zusammenbau hineingleiten können, weisen die Blindlöcher 9 der Deckplatte 6 kegelförmige Ansenkungen 25 der Ränder 29 der Blindlöcher 9 auf. Ferner besitzt die Deckplatte Durchgangslöcher 27, die sich im Mittelpunkt der Dreiecke befinden, die von drei Blindlöchern 9 der Deckplatte 6 jeweils gebildet werden.

Figur 4 zeigt eine schematische perspektivische Ansicht des Einsatzes 4 in Bestrahlungsposition, dazu ist der zusammengebaute Einsatz 4 aus stabförmigen Zellträgern 3, Bodenplatte 5, Deckplatte 6, Abstandshalter 16 und Standbeinen 28 um 180° gedreht, so dass nun die Deckplatte 6 den unteren Zellträgerhalter darstellt und durch die in der Deckplatte 6 angeordneten Durchgangslöcher Gas, beispielsweise Sauerstoff und/oder Kohlendioxid und Nährstoff strömen kann, um die lebenden Zellen auf den Mantelflächen 13 der stabförmigen Zellträgern 3 zu versorgen. Die Bodenplatte 5 des Zusammenbaus bildet nun in dem Behälter 2, dessen Kontur mit gestrichelter Linie angedeutet ist, den oberen Zellträgerhalter, während sich die Standbeine 28 auf dem Innenboden 30 des Behälters 2 abstützen und einen Zwischenraum zum Innenboden 30 bilden, in dem diese Ausführungsform der Erfindung 5 nicht gezeigte Rührsysteme, die mit 100 bis 200 Umdrehungen pro Minute, vorzugsweise mit 130 Umdrehungen pro Minute arbeiten, angeordnet sind.

Die stabförmigen Zellträger 3 sind in 24 Reihen angeordnet und zu 23 bzw. 24 Vollstäben 12 mit Zellmaterial auf dem Mantelflächen 13 versetzt zueinander zwischen den Zellträgerhaltern 5 und 6 bzw. zwischen den Halteplatten 5 und 6 angeordnet. Um einen vorgegebenen Abstand zwischen den Platten 5 und 6 einzuhalten, ohne die stabförmigen Zellträger 3 zu belasten sind in vier Vorsprüngen 17-20 Sacklöcher mit einem Durchmesser von 8 mm und einer mittigen Durchgangsbohrung von 3,2 mm angebracht, so dass Hohlzylinder als Abstandshalter 16 mit Innengewinde eingesetzt werden können, so dass der obere Zellträgerhalter 5 mit aus Nylon bestehenden Gewindestangen oder Schrauben an dem Abstandshalter fixiert werden kann, und der untere Zellträger 6 über Gewindestangen und die auf die Gewindestangen verschraubten Standbeinen 30 verbunden ist. Durch die Abstandshalter 16 mit Innengewindestange wurde somit ein präzises Gestell zur örtlichen Fixierung der Zellträger relativ zum Boden des Behälters geschaffen, wobei die Standbeine in vorgefertigte nicht gezeigte Elemente auf dem Boden 30 des Behälters 2 eingeführt werden können, um eine genaue Justierung der Zellträger 3 in Bezug auf die Strahlrichtung Z der Bestrahlungsanlage in der Bestrahlungsposition vorzunehmen.

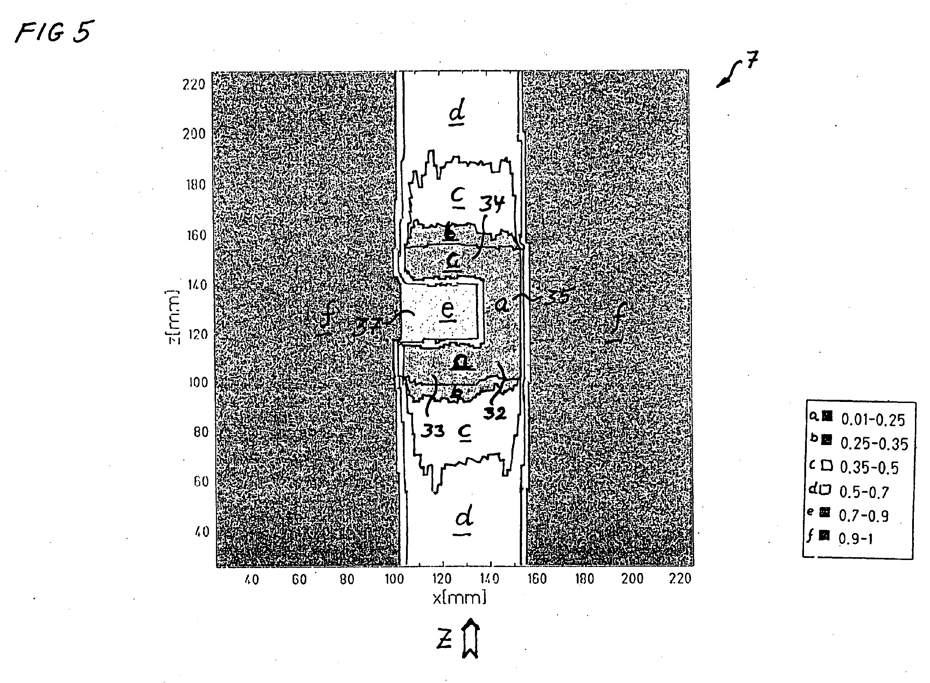

Figur 5 zeigt ein geplantes Bestrahlungskonzept 7, dass im folgenden mit zwei Bestrahlungsverifikations-Experimenten und mit Hilfe der erfindungsgemäßen Bestrahlungsverifikationsvorrichtung untersucht wird, dabei zeigt der U-förmige Bereich 32 des hier aufgezeigten Bestrahlungskonzepts 7 einen Schnitt durch ein Tumorvolumen, wobei die Strahlrichtung Z in z-Richtung ist, und es bei diesem Bestrahlungsplan 7 darauf ankommt, dass in einer Tiefe von etwa 90-110 mm in z-Richtung ein möglichst hohes Absterben des Zellmaterials erreicht werden soll, und dieses Absterben in z-Richtung zwischen 110 und 140 mm in einer Breite zwischen 100 und 140 mm in horizontaler Ablenkrichtung möglichst gering beschädigt oder gar nicht beschädigt werden soll und in einer weiteren Tiefe zwischen 140 und 160 mm wiederum Zellmaterial beschädigt werden soll. Demgegenüber soll in einem horizontalen Ablenkbereich zwischen etwa 140 mm und 155 mm durchgängig in einer Tiefe zwischen etwa 90 mm und 160 mm das gesamte Zellmaterial beschädigt werden. Die Buchstaben a bis f geben Überlebensraten der Zellmaterialien in den geplanten Bereichen an.

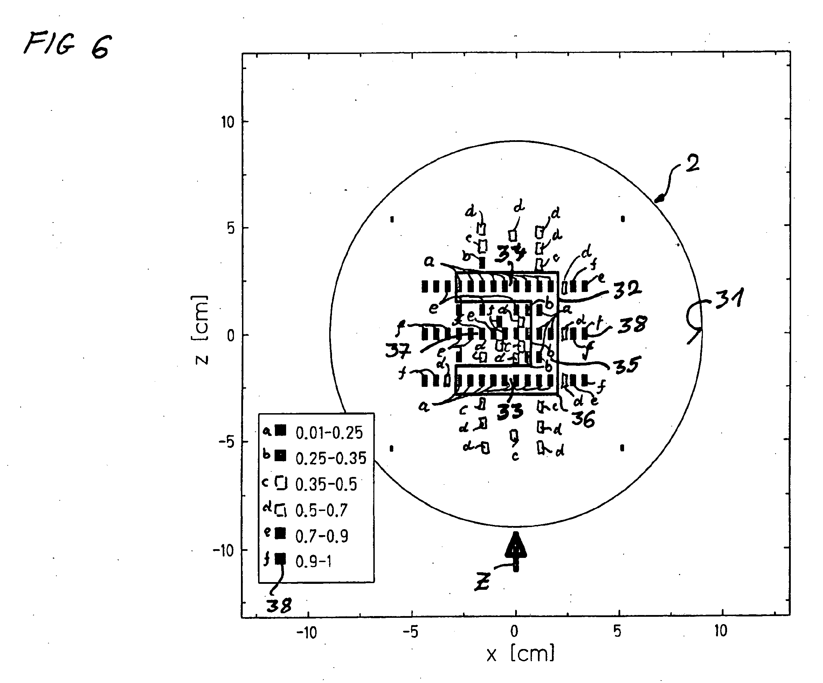

Figur 6 zeigt eine schematische Draufsicht auf den Behälter 2 der Bestrahlungsverifikationsvorrichtung, wobei hier lediglich die Grenze der Innenwandung 31 als Kreislinie gezeigt wird, und der geplante u-förmige Bereich 32 mit zwei Schenkeln 33 und 34 und dem Verbindungsstück 35 mit einer Konturlinie 36 dargestellt wird. Die Strahlrichtung ist durch den Pfeil Z gezeigt, während die Zahlenwerte die Überlebensraten des Zellmaterials angeben. Bereits mit dem ersten Experiment kann mit der Bestrahlungsverifikationsvorrichtung deutlich gezeigt werden, dass die Überlebensrate im Bereich der Schenkel 33 und 34 und im Bereich des Verbindungsstücks 35 mit 0,01 bis 0,25 äußerst gering ist, während in dem dazwischen liegendem Tiefenbereich 37 die Überlebenschancen des Zellmaterial mit Überlebensraten zwischen 0,7 bis 1,5 deutlich höher liegen, obgleich auch dieser Zwischenbereich 37 zwischen den Bestrahlungsschenkeln 33 und 34 einer Strahlenbelastung ausgesetzt ist, da die Ionen, die den tieferliegenden Schenkel 34 des U-förmigen Bestrahlungsbereichs 32 erreichen sollen, diesen Zwischenbereich 37 durchstrahlen müssen.

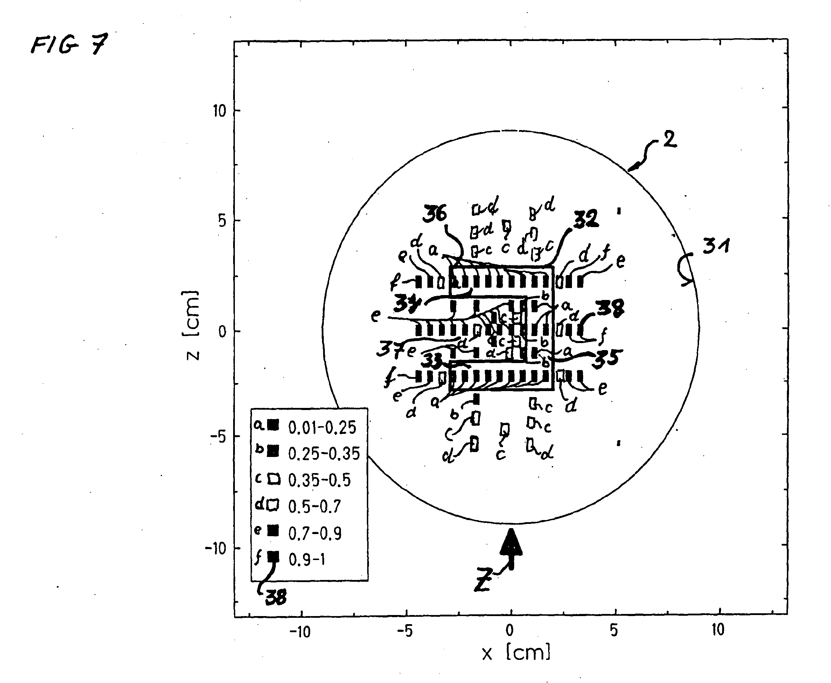

Figur 7 zeigt eine schematische Draufsicht auf den Behälter 2 der Bestrahlungsverifikationsvorrichtung mit Ergebnissymbolen 38 für Gruppen von Zellträgern, mit denen das erste Ergebnis bestätigt wird, dass in dem U-förmigen Bereich Zellen mit einer Überlebensrate in der Größenordnung von 0,01 bis 0,25 überlebt haben, während das außerhalb des U-förmigen Bestrahlungsbereichs 32 angeordnete Zellmaterial deutlich weniger beschädigt ist.

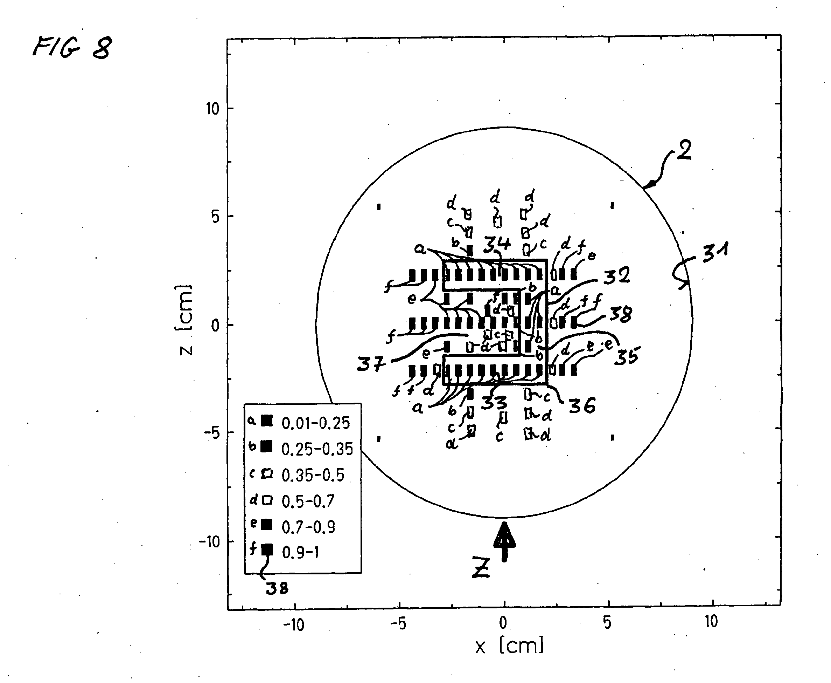

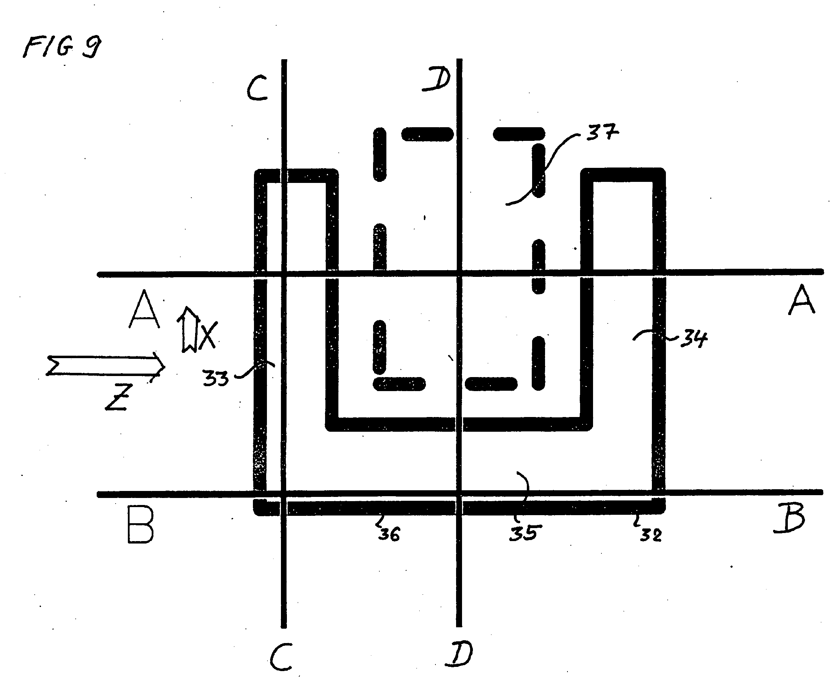

Figur 8 zeigt eine schematische Draufsicht auf einen Behälter 2 der Bestrahlungsverifikationsvorrichtung 1 mit gemittelten Ergebnissymbolen 38 für Gruppen von Zellträgern 3. Durch die Mittelung der Messergebnisse hebt sich der U-förmige stärker bestrahlte Bereich auch im Ergebnis der gemittelten überlebensraten des Zellmaterials deutlich ab. Eine genauere Analyse der Ergebnisse liefern die Figuren 9 bis 13.

Figur 9 zeigt einen Plan der Lage von Verifikationsmesslinien durch den U-förmigen Bestrahlungsbereich 32, wobei die Messlinien A-A und B-B der Strahlrichtung Z bzw. der Strahlachse folgen und die Messlinien C-C und D-D in der horizontalen Auslenkrichtung X des Strahls aufgenommen sind.

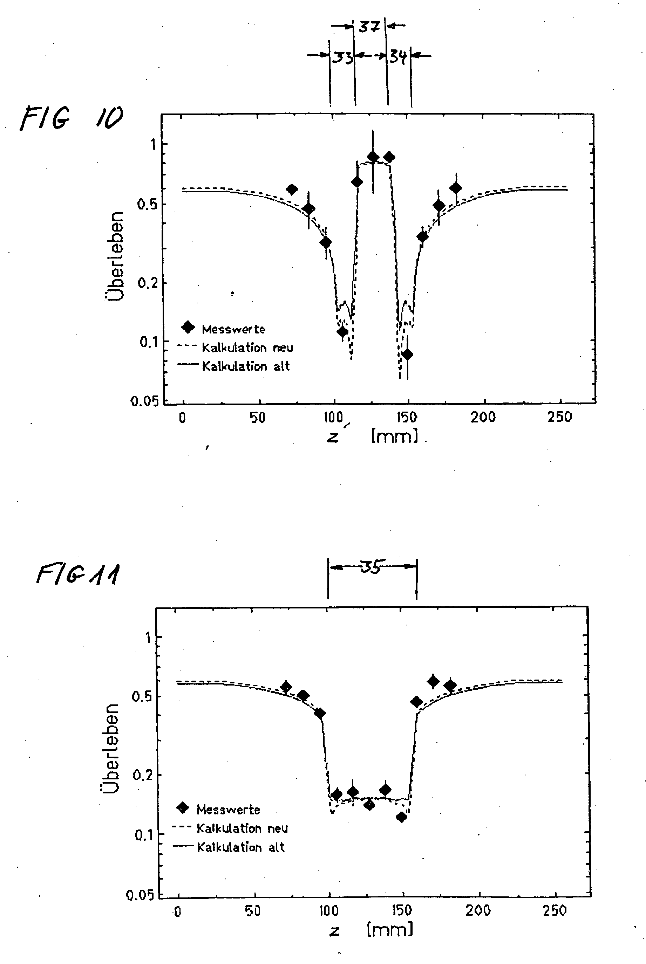

Figur 10 zeigt Mittelwerte von Messergebnissen entlang der Verifikationsmesslinie A-A in Figur 9. Während die Verifikationsmesslinie A-A durch die Schenkel 33 und 34 des U-förmigen Bereichs sowie durch das dazwischen liegende Gebiet 37 verläuft, ist die Verifikationsmesslinie B-B im Verbindungsstück des U-förmigen Bereichs angeordnet. Der Mittelwert, der in Figur 10 dargestellt wird, zeigt deutlich, dass die Überlebensrate in dem Zwischenbereich 37 deutlich höher liegt als in den Bereichen vor und nach dem U-förmigen Bereich liegt. Außerdem zeigen die Messwerte in den Schenkelbereichen 33 und 34, dass die Überlebenschancen des Zellmaterials, das entlang der Ordinate aufgetragen ist, deutlich geringer ist als im Zwischenbereich 37 zwischen den Schenkeln 33 und 34.

Figur 11 zeigt die Mittelwerte von Messergebnissen entlang der Verifikationsmesslinie B-B in Figur 9, bei dem in z-Richtung das Verbindungsstück 35 des U-förmigen Bereichs 32 untersucht wird und die Messwerte des Überlebens zeigen deutlich, dass in diesem Bereich das überlebende Zellmaterial in der ganzen Breite des Zwischenstücks 35 deutlich absinkt.

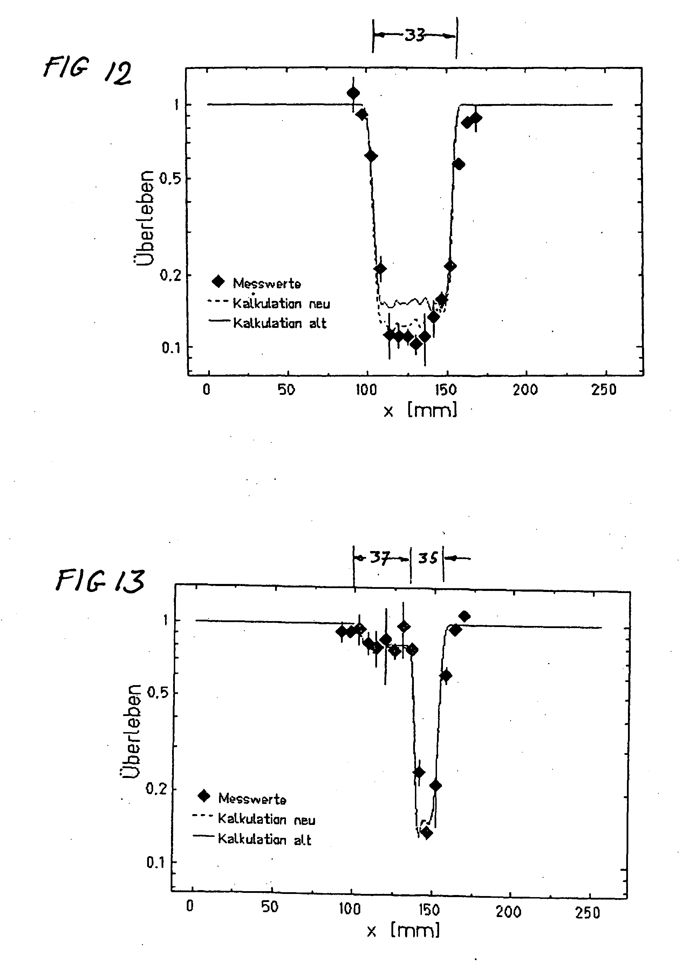

Figur 12 zeigt Mittelwerte von Messergebnissen entlang der Verifikationsmesslinie C-C in Figur 9, die vollständig durch den vorderen zum Strahleintritt hin gelegenen Schenkel gelegt ist, und die Werte in der horizontalen Ablenkrichtung x zeigt. Die mit Hilfe von Bragg'schen Abbremskurven für Ionen theoretisch kalkulierten Werte, die mit durchgezogener Linie gezeichnet sind, werden von den Messwerten deutlich im Schenkelbereich 33 unterschritten, wobei die effektive Inaktivierung der Tumorzellen erhöht wird.

Figur 13 zeigt Mittelwerte von Messergebnissen entlang der Verifikationsmesslinie D-D in Figur 9, bei der zunächst der Zwischenbereich 37 zwischen den beiden Schenkeln 33 und 34 gemessen wird und anschließend das Verbindungsstück in x-Richtung der horizontalen Auslenkrichtung X des Stahls durchquert wird, wobei ein schwaches Absinken der Überlebenschance des Zellmaterials im Zwischenbereich zu beobachten ist. Erst innerhalb des Verbindungsstücks 35 wird ein deutliches Absinken des Überlebens des Zellmaterials nachgewiesen. Die durchgezogene Linie zeigt wieder die berechneten Werte aufgrund der Bragg'schen Abbremskurven für Ionen in Geweben.

### Bezugszeichenliste

- 1: Bestrahlungsverifikationsvorrichtung
- 2: Behälter
- 3: Zellträger
- 4: Einsatz
- 5: Bodenplatte bzw. Halteplatte
- 6: Deckplatte bzw. Halteplatte
- 7: Bestrahlungskonzept
- 8: Blindloch (Bodenplatte)
- 9: Blindloch (Deckplatte)
- 10: Ende des Zellträgers (Bodenplatte)
- 11: Enden des Zellträgers (Deckplatte)
- 12: Vollstab
- 13: Mantelfläche
- 14: Reihe von Zellträgern
- 15: Reihe von Zellträgern
- 16: Abstandshalter
- 17: Vorsprung
- 18: Vorsprung
- 19: Vorsprung
- 20: Vorsprung
- 21: Randseite
- 22: Randseite
- 23: Randseite
- 24: Randseite
- 25: Kegelförmige Ansenkung (Bodenplatte)
- 26: Kegelförmige Ansenkung (Deckplatte)
- 27: Durchgangsloch
- 28: Standbein
- 29: Rand eines Blindloches
- 30: Innenboden des Behälters
- 31: Innenwandung
- 32: u-förmiger Bereich
- 33: Schenkel des u-förmigen Bereichs
- 34: Schenkel des u-förmigen Bereichs
- 35: Verbindungsstück des u-förmigen Bereichs
- 36: Konturlinie
- 37: Zwischenbereich

- A-A: Verifikationsmesslinie
- B-B: Verifikationsmesslinie
- C-C: Verifikationsmesslinie
- D-D: Verifikationsmesslinie
- t: Tiefe eines Blindlochs
- h: Höhe eines Standbeins
- d: Dicke der Bodenplatte
- Z: Strahlrichtung
- X: horizontale Auslenkrichtung des Strahls
- Y: vertikale Auslenkrichtung des Strahls
- x y z: Raumkoordinaten

## Patentansprüche

1. Bestrahlungsverifikationsvorrichtung für Strahlentherapieanlagen, wobei die Bestrahlungsverifikationsvorrichtung (1) lebendes Zellmaterial aufweist, das in den drei Raumkoordinaten x, y und z in einem Behälter (2) mit einem Einsatz (4) auf Zellträgern (3) des Einsatzes (4) örtlich fixiert ist, wobei die Zellträger (3) zwischen Zellträgerhaltern (5,6) angeordnet sind und wobei zur Bestrahlungsverifikation die z-Koordinate der Bestrahlungsverifikationsvorrichtung (1) in Richtung der Strahlachse Z justiert ist, so dass nach der Bestrahlung Bereiche mit abgestorbenem Zellmaterial von Bereichen mit noch aktivem Zellmaterial der Bestrahlungsverifikationsvorrichtung (1) in Raumkoordinaten unter Bezug auf ein Bestrahlungskonzept (7) lokalisierbar sind, wobei
- der Behälter (2) mit dem Einsatz (4) und die Zellträger (3) sowie die Zellträgerhalter (5,6) ein strahlendurchlässiges Material aufweisen,
- die Zellträgerhalter (5,6) eine Bodenplatte (5) und eine der Bodenplatte (5) gegenüberliegende Deckplatte (6) aufweisen, zwischen denen die Zellträger (3) orthogonal zu den Halteplatten (5,6) angeordnet sind,
**dadurch gekennzeichnet, dass** die Halteplatten (5,6) aufeinander ausgerichtete Blindlöcher (8,9) aufweisen, in denen die Enden (10, 11) der Zellträger (3) angeordnet sind und die Zellträger (3) kreiszylindrische Vollstäbe (12) sind, auf deren Mantelflächen (13) das Zellmaterial fixiert ist.

2. Bestrahlungsverifikationsvorrichtung nach Anspruch 1, **dadurch gekennzeichnet, dass** die Zellträger (3) weder Metalle noch Elemente höherer Massenzahl als Sauerstoff aufweisen, eine wassernahe Dichte von etwa 1 g/cm³ besitzen und vorzugsweise Polystyrol umfassen.

3. Bestrahlungsverifikationsvorrichtung nach Anspruch 1 oder 2, **dadurch gekennzeichnet, dass** die Zellträger (3) versetzt zueinander zwischen den Halteplatten (5,6) in Reihen (14,15) angeordnet sind.

4. Bestrahlungsverifikationsvorrichtung nach einem der Ansprüche 1 bis 3, **dadurch gekennzeichnet, dass** die Zellträger (3) eine Länge 1 von 25, mm ≤ l ≤ 75 mm vorzugsweise etwa 50 mm aufweisen.

5. Bestrahlungsverifikationsvorrichtung nach einem der Ansprüche 1 bis 4, **dadurch gekennzeichnet, dass** die Zellträger (3) einen Durchmesser d in Millimetern zwischen 1,0 mm ≤ d ≤ 5 mm, insbesondere 1,5 mm ≤ d ≤ 5 mm vorzugsweise etwa 3 mm aufweisen.

6. Bestrahlungsverifikationsvorrichtung nach einem der Ansprüche 1 bis 5 **dadurch gekennzeichnet, dass** der minimale Abstand a zwischen den Zellträgern (3) in Millimetern zwischen 0,5 mm ≤ a ≤ 5 mm, vorzugsweise 2,5 mm, aufweist.

7. Bestrahlungsverifikationsvorrichtung nach einem der Ansprüche 1 bis 6, **dadurch gekennzeichnet, dass** zwischen den Halteplatten (5,6) mindestens drei, vorzugsweise vier, Abstandshalter (16) angeordnet sind.

8. Bestrahlungsverifikationsvorrichtung nach einem der Ansprüche 1 bis 7, **dadurch gekennzeichnet, dass** die Abstandshalter (16) zwischen Vorsprüngen (17-20) auf Randseiten (21-24) der Halteplatten (5) angeordnet sind.

9. Bestrahlungsverifikationsvorrichtung nach einem der Ansprüche 1 bis 7, **dadurch gekennzeichnet, dass** die Bodenplatte (5) tiefere Blindlöcher (8) zum Fixieren eines Endes (10) der Vollstäbe (12) als die Deckplatte (6) aufweist.

10. Bestrahlungsverifikationsvorrichtung nach einem der Ansprüche 1 bis 9, **dadurch gekennzeichnet, dass** die Blindlöcher (8,9) der Halteplatten (5,6) eine kegelförmige Ansenkung (25,26) des Außenrandes aufweisen.

11. Bestrahlungsverifikationsvorrichtung nach einem der Ansprüche 1 bis 10, **dadurch gekennzeichnet, dass** mindestens eine der Halteplatten (5,6) zwischen den Blindlöchern (8,9) auf der Oberseite verteilte Durchgangslöcher (27) aufweist.

12. Bestrahlungsverifikationsvorrichtung nach einem der Ansprüche 1 bis 9, **dadurch gekennzeichnet, dass** die Halteplatten (5,6) Markierungen zur Kennzeichnung der Positionen der Zellträger (3) aufweisen.

13. Bestrahlungsverifikationsvorrichtung nach einem der Ansprüche 1 bis 12, **dadurch gekennzeichnet, dass** der Einsatz (4) Standbeine (28) zum Bodenbereich des Behälters (2) aufweist.

14. Bestrahlungsverifikationsvorrichtung nach einem der Ansprüche 1 bis 13, **dadurch gekennzeichnet, dass** der Behälter (2) eine Einlassöffnung und eine Auslassöffnung für eine Nährflüssigkeit und/oder für Gas(e), insbesondere für Sauerstoff aufweist.

15. Bestrahlungsverifikationsvorrichtung nach einem der Ansprüche 1 bis 14, **dadurch gekennzeichnet, dass** der Behälter (2) eine zylindrische Innenwand und im Bodenbereich mindestens eine Rührvorrichtung zur Verteilung von Nährflüssigkeit zu den lebenden Zellmaterialien auf den Mantelflächen (13) der stabförmigen Zellträger (3) aufweist.

16. Bestrahlungsverifikationsvorrichtung nach einem der Ansprüche 1 bis 12, **dadurch gekennzeichnet, dass** die Bestrahlungsverifikationsvorrichtung (1) in Richtung der y-Raumkoordinate eine höhere Ortsauflösung für eine Y-Auslenkrichtung des Strahls aufweist, als in Richtung der x-Raumkoordinate für die X-Auslenkrichtung des Strahls.

17. Bestrahlungsverifikationsvorrichtung nach einem der Ansprüche 1 bis 12, **dadurch gekennzeichnet, dass** die Bestrahlungsverifikationsvorrichtung (1) in Richtung der z-Raumkoordinate die gleiche Ortsauflösung für die Z-Ausbreitungsrichtung des Strahls aufweist, wie in Richtung der x-Raumkoordinate für die X-Auslenkrichtung des Strahls.

18. Bestrahlungsverifikationsvorrichtung nach einem der Ansprüche 1 bis 12, **dadurch gekennzeichnet, dass** die Bestrahlungsverifikationsvorrichtung (1) eine Anzahl S von Zellträgern (3) von 150 ≤ S ≤ 900 und insbesondere 400 ≤ S ≤ 900 aufweist.

19. Verwendung der Bestrahlungsverifikationsvorrichtung (1) gemäß einem der vorhergehenden Ansprüche in Strahlentherapieanlagen, wie Röntgenstrahltherapieanlagen oder Protonenstrahltherapieanlagen insbesondere in Heliumionenstrahltherapieanlagen oder Kohlenstoffionenstrahltherapieanlagen.

20. Verfahren zur Handhabung der Bestrahlungsverifikationsvorrichtung (1) gemäß einem der Ansprüche 1-18, wobei das Verfahren nachfolgende Verfahrensschritte aufweist:
- Belegen der Mantelflächen (13) der stabförmigen Zellträgern (3) mit aktivem lebendem Zellmaterial,
- Zusammenbau eines Einsatzes (4) eines Behälters (2) der Bestrahlungsverifikationsvorrichtung (1) unter Anordnen der Zellträger (3) mit lebendem Zellmaterial zwischen zwei Halteplatten (5,6) des Einsatzes (4) und Sichern des zusammengebauten Einsatzes (4) über Abstandshalter (16),
- Einbringen des Einsatzes (4) in den zwischenzeitlich mit Nährflüssigkeit aufgefüllten Behälter (2),
- Zuführen von Gas(en), insbesondere von Sauerstoff über eine Einlassöffnung in den Behälter (2),
- Ausrichten der x-, y-, und z-Raumkoordinaten der Bestrahlungsverifikationsvorrichtung (1) zur Strahlrichtung,
- Durchführen eines geplanten Bestrahlungskonzepts (7) unter gleichzeitiger oder nachträglicher Temperierung der Nährflüssigkeit, um die Ergebnisse zu konservieren,
- Ausbau der Zellträger (3) aus dem Einsatz (4),
- Auftrennen der stabförmigen Zellträger (3) in Scheiben und Detektieren des noch lebenden Zellmaterials auf den getrennten Scheiben,
- Vergleichen der Ergebnisse in Bezug auf die gewählten analytischen Endpunkte und den Bestrahlungsplan.

## Claims

1. Irradiation verification apparatus for beam therapy systems, the irradiation verification apparatus (1) containing living cell material which is fixed in location in the three spatial co-ordinates x, y and z in a container (2) having an insert (4), on cell supports (3) of the insert (4), the cell supports (3) being arranged between cell support holders (5,6) and, for irradiation verification, the z co-ordinate of the irradiation verification apparatus (1) being adjusted in the direction of the beam axis Z so that, after the irradiation, regions having killed cell material can be demarcated in spatial co-ordinates, with reference to an irradiation scheme (7), from regions having cell material of the irradiation verification apparatus (1) that is still active, wherein
- the container (2) having the insert (4) and the cell supports (3) and the cell support holders (5,6) comprise a beam-transparent material,
- the cell support holders (5,6) have a base plate (5) and a top plate (6) located opposite the base plate (5), between which the cell supports (3) are arranged orthogonally to the holding plates (5,6),
**characterised in that** the holding plates (5, 6) having blind holes (8, 9) aligned with one another, in which the ends (10, 11) of the cell supports (3) are arranged, and the cell supports (3) are circular cylindrical solid rods (12), on the outer surfaces (13) of which the cell material is fixed.

2. Irradiation verification apparatus according to claim 1, **characterised in that** the cell supports (3) comprise neither metals nor elements having a higher atomic mass number than oxygen, have a density close to water of about 1 g/cm³ and preferably comprise polystyrene.

3. Irradiation verification apparatus according to claim 1 or 2, **characterised in that** the cell supports (3) are arranged between the holding plates (5,6) in rows (14,15) offset from one another.

4. Irradiation verification apparatus according to one of claims 1 to 3, **characterised in that** the cell supports (3) have a length 1 of 25 mm ≤ l ≤ 75 mm, preferably about 50 mm.

5. Irradiation verification apparatus according to one of claims 1 to 4, **characterised in that** the cell supports (3) have a diameter d in millimetres between 1.0 mm ≤ d ≤ 5 mm, especially 1.5 mm ≤ d ≤ 5 mm, preferably about 3 mm.

6. Irradiation verification apparatus according to one of claims 1 to 5, **characterised in that** the minimum spacing a in millimetres between the cell supports (3) is between 0.5 mm ≤ a ≤ 5 mm, preferably 2.5 mm.

7. Irradiation verification apparatus according to one of claims 1 to 6, **characterised in that** between the holding plates (5,6) there are arranged at least three, preferably four, spacers (16).

8. Irradiation verification apparatus according to one of claims 1 to 7, **characterised in that** the spacers (16) are arranged between projections (17-20) on peripheral sides (21-24) of the holding plates (5).

9. Irradiation verification apparatus according to one of claims 1 to 7, **characterised in that** the base plate (5) has deeper blind holes (8) for fixing one end (10) of the solid rods (12) than the top plate (6).

10. Irradiation verification apparatus according to one of claims 1 to 9, **characterised in that** the blind holes (8,9) of the holding plates (5,6) have a tapered countersink (25,26) of the outer edge.

11. Irradiation verification apparatus according to one of claims 1 to 10, **characterised in that** at least one of the holding plates (5,6) has through-holes (27) distributed between the blind holes (8,9) on the upper side.

12. Irradiation verification apparatus according to one of claims 1 to 9, **characterised in that** the holding plates (5,6) have markings for identifying the positions of the cell supports (3).

13. Irradiation verification apparatus according to one of claims 1 to 12, **characterised in that** the insert (4) has standing legs (28) directed to the base region of the container (2).

14. Irradiation verification apparatus according to one of claims 1 to 13, **characterised in that** the container (2) has an inlet aperture and an outlet aperture for a nutrient liquid and/or for gas(es), especially oxygen.

15. Irradiation verification apparatus according to one of claims 1 to 14, **characterised in that** the container (2) has a cylindrical inner wall and, in the base region, at least one stirring apparatus for the distribution of nutrient liquid to the living cell material on the outer surfaces (13) of the rod-shaped cell supports (3).

16. Irradiation verification apparatus according to one of claims 1 to 12, **characterised in that** the irradiation verification apparatus (1) has, in the direction of the y spatial co-ordinate, a higher location resolution for a Y deflection direction of the beam than in the direction of the x spatial co-ordinate for the X deflection direction of the beam.

17. Irradiation verification apparatus according to one of claims 1 to 12, **characterised in that** the irradiation verification apparatus (1) has, in the direction of the z spatial co-ordinate, the same location resolution for the Z propagation direction of the beam as in the direction of the x spatial co-ordinate for the X deflection direction of the beam.

18. Irradiation verification apparatus according to one of claims 1 to 12, **characterised in that** the irradiation verification apparatus (1) has a number S of cell supports (3) of 150 ≤ S ≤ 900, especially 400 ≤ S ≤ 900.

19. Use of the irradiation verification apparatus (1) according to one of the preceding claims in beam therapy systems such as X-ray beam therapy systems or proton beam therapy systems, especially in helium ion beam therapy systems or carbon ion beam therapy systems.

20. Method for the operation of the irradiation verification apparatus (1) according to one of claims 1 to 18, wherein the method comprises the following method steps:
- providing the outer surfaces (13) of the rod-shaped cell supports (3) with active living cell material,
- assembly of an insert (4) of a container (2) of the irradiation verification apparatus (1), with arrangement of the cell supports (3) having living cell material between two holding plates (5,6) of the insert (4) and securing of the assembled insert (4) by means of spacers (16),
- introducing the insert (4) into the container (2), which in the meantime has been filled with nutrient liquid,
- supplying gas(es), especially oxygen, by way of an inlet aperture, to the container (2),
- orienting the x, y and z spatial co-ordinates of the irradiation verification apparatus (1) relative to the beam direction,
- implementing a planned irradiation scheme (7), with simultaneous or subsequent temperature control of the nutrient liquid in order to preserve the results,
- disassembly of the cell supports (3) from the insert (4),
- separating the rod-shaped cell supports (3) into slices and detection of the still living cell material on the separated slices,
- comparing the results with reference to the selected analytical end-points and the irradiation plan.

## Revendications

1. Dispositif de vérification d'irradiation pour des installations de radiothérapie, ledit dispositif de vérification d'irradiation (1) comportant un matériau cellulaire vivant, qui est fixé localement dans les trois coordonnées spatiales x, y et z dans un récipient (2) avec un insert (4), sur des supports cellulaires (3) de l'insert (4), les supports cellulaires (3) étant disposés entre des plaques à supports cellulaires (5, 6), et la coordonnée z du dispositif de vérification d'irradiation (1) étant ajustée dans la direction de l'axe de rayonnement z pour la vérification d'irradiation, de sorte qu'après irradiation, des zones avec du matériau cellulaire nécrosé puissent être localisées dans les coordonnées spatiales par rapport à des zones avec du matériau cellulaire encore actif du dispositif de vérification d'irradiation (1), en référence à un concept d'irradiation (7), où
- le récipient (2) avec l'insert (4) et les supports cellulaires (3) ainsi que les plaques à supports cellulaires (5, 6) comportent un matériau perméable aux rayons,
- les plaques à supports cellulaires (5, 6) comportent une plaque de base (5) et une plaque de sommet (6) opposée à la plaque de base (5), entre lesquelles les supports cellulaires (3) sont disposés orthogonalement aux plaques de maintien (5, 6),
**caractérisé en ce que** les plaques de maintien (5, 6) comportent des trous borgnes (8, 9) opposés les uns aux autres, où sont logées les extrémités (10, 11) des supports cellulaires (3), et **en ce que** les supports (3) sont des tiges cylindriques circulaires pleines (12), sur les surfaces d'enveloppe (13) desquelles le matériau cellulaire est fixé.

2. Dispositif de vérification d'irradiation selon la revendication 1, **caractérisé en ce que** les supports cellulaires (3) ne comprennent ni métaux, ni éléments de masse atomique supérieure à l'oxygène, présentant une densité proche de l'eau de quelque 1 g/cm³ et comprenant de préférence du polystyrène.

3. Dispositif de vérification d'irradiation selon la revendication 1 ou la revendication 2, **caractérisé en ce que** les supports cellulaires (3) sont disposés en rangées (14, 15) décalées les unes par rapport aux autres entre les plaques de maintien (5, 6).

4. Dispositif de vérification d'irradiation selon l'une des revendications 1 à 3, **caractérisé en ce que** les supports cellulaires (3) présentent une longueur 1 telle que : 25 mm ≤ l ≤ 75 mm, de préférence égale à 50 mm environ.

5. Dispositif de vérification d'irradiation selon l'une des revendications 1 à 4, **caractérisé en ce que** les supports cellulaires (3) présentent un diamètre d mesuré en mm, tel que : 1,0 mm ≤ d ≤ 5 mm, en particulier 1,5 mm ≤ d ≤ 5 mm, de préférence égal à 3 mm environ.

6. Dispositif de vérification d'irradiation selon l'une des revendications 1 à 5, **caractérisé en ce que** l'espacement minimal a mesuré en mm entre les supports cellulaires (3) est tel que : 0,5 mm ≤ a ≤ 5 mm, de préférence égal à 2,5 mm.

7. Dispositif de vérification d'irradiation selon l'une des revendications 1 à 6, **caractérisé en ce qu'**au moins trois, de préférence quatre écarteurs (16) sont disposés entre les plaques de maintien (5, 6).

8. Dispositif de vérification d'irradiation selon l'une des revendications 1 à 7, **caractérisé en ce que** les écarteurs (16) sont disposés entre des parties en saillie (17-20) sur les côtés (21-24) des plaques de maintien (5).

9. Dispositif de vérification d'irradiation selon l'une des revendications 1 à 7, **caractérisé en ce que** la plaque de base (5) comporte des trous borgnes (8) plus profonds que ceux de la plaque de sommet (6) pour la fixation d'une extrémité (10) des tiges pleines (12).

10. Dispositif de vérification d'irradiation selon l'une des revendications 1 à 9, **caractérisé en ce que** les trous borgnes (8, 9) des plaques de maintien (5, 6) sont pourvus d'un chanfrein conique (25, 26) sur leur bord extérieur.

11. Dispositif de vérification d'irradiation selon l'une des revendications 1 à 10, **caractérisé en ce qu'**au moins une des plaques de maintien (5, 6) comporte des trous débouchants (27) répartis entre les trous borgnes (8, 9) sur sa face supérieure.

12. Dispositif de vérification d'irradiation selon l'une des revendications 1 à 9, **caractérisé en ce que** les plaques de maintien (5, 6) présentent des marques pour la reconnaissance de position des supports cellulaires (3).

13. Dispositif de vérification d'irradiation selon l'une des revendications 1 à 12, **caractérisé en ce que** l'insert (4) comporte des jambes de soutien (28) vers la zone de fond du récipient (2).

14. Dispositif de vérification d'irradiation selon l'une des revendications 1 à 13, **caractérisé en ce que** le récipient (2) comporte une ouverture d'admission et une ouverture d'évacuation pour un liquide nutritif et/ou pour un/des gaz, en particulier de l'oxygène.

15. Dispositif de vérification d'irradiation selon l'une des revendications 1 à 14, **caractérisé en ce que** le récipient (2) comporte une paroi intérieure cylindrique et dans la zone de fond au moins un dispositif agitateur pour la répartition de liquide nutritif vers les matériaux cellulaires vivants sur les surfaces d'enveloppe (13) des supports cellulaires (3) en forme de tiges.

16. Dispositif de vérification d'irradiation selon l'une des revendications 1 à 12, **caractérisé en ce que** ledit dispositif de vérification d'irradiation (1) présente dans la direction de la coordonnée spatiale y une résolution locale supérieure pour une direction de déviation Y du faisceau, qu'en direction de la coordonnée spatiale x pour la direction de déviation X du faisceau.

17. Dispositif de vérification d'irradiation selon l'une des revendications 1 à 12, **caractérisé en ce que** ledit dispositif de vérification d'irradiation (1) présente dans la direction de la coordonnée spatiale z la même résolution locale pour la direction de diffusion Z du faisceau, qu'en direction de la coordonnée spatiale x pour la direction de déviation X du faisceau.

18. Dispositif de vérification d'irradiation selon l'une des revendications 1 à 12, **caractérisé en ce que** ledit dispositif de vérification d'irradiation (1) présente un nombre S de supports cellulaires (3) tel que 150 ≤ S ≤ 900, et en particulier 400 ≤ S ≤ 900.

19. Utilisation du dispositif de vérification d'irradiation (1) selon l'une des revendications précédentes dans des installations de radiothérapie telles que des installations de radiothérapie à rayons X ou des installations de radiothérapie protonique, en particulier dans des installations de radiothérapie à ions hélium ou des installations de radiothérapie à ions carbone.

20. Procédé pour la manipulation du dispositif de vérification d'irradiation (1) selon l'une des revendications précédentes, ledit procédé comprenant les étapes suivantes :
- couverture des surfaces d'enveloppe (13) de supports cellulaires en forme de tiges (3) avec du matériau cellulaire actif,
- montage d'un insert (4) d'un récipient (2) du dispositif de vérification d'irradiation (1) par disposition des supports cellulaires (3) avec du matériau cellulaire vivant entre deux plaques de maintien (5, 6) de l'insert (4) et fixation de l'insert (4) monté au moyen d'écarteurs (16),
- mise en place de l'insert (4) dans le récipient (2) préalablement rempli de liquide nutritif,
- alimentation en gaz, en particulier de l'oxygène, par une ouverture d'admission du récipient (2),
- ajustement des coordonnées spatiales x, y et z du dispositif de vérification d'irradiation (1) vers la direction de rayonnement,
- exécution d'un concept d'irradiation (7) projeté par thermostatisation simultanée ou ultérieure du liquide nutritif, afin de conserver les résultats,
- démontage des supports cellulaires (3) de l'insert (4),
- découpage des supports cellulaires (3) en rondelles et détection du matériau cellulaire encore vivant sur les rondelles découpées,
- comparaison des résultats par rapport aux critères d'évaluation analytiques sélectionnés et au plan d'irradiation.
